# EUROPEAN PATENT SPECIFICATION

(11) **EP 1 767 237 B1**
(45) Date of publication and mention of the grant of the patent: **24.10.2012**
(21) Application number: 06254875.5
(22) Date of filing: 20.09.2006
(51) Int. Cl.: A61M 16/00

(54) **Method and device for the operation of an artificial respiration system**
Verfahren und Vorrichtung zur Steuerung eines Beatmungsgerät
Procédé et dispositif pour l'opération d'un appareil de respiration artificielle

(30) Priority: 24.09.2005 DE 102005045720
(43) Date of publication of application: 28.03.2007
(73) Proprietor: Dräger Medical GmbH, 23558 Lübeck (DE)
(72) Inventor: Schermeier, Olaf, 23560 Lübeck (DE); Klessaschek, Thomas, 23558 Lübeck (DE); Zelk, Marco, 23619 Hamberge (DE); Kellner, Bernd, 23570 Lübeck (DE)
(74) Representative: UEXKÜLL & STOLBERG

(56) References cited:
- EP-A- 1 731 089
- WO-A-02/078775
- WO-A-2005/002655
- DE-U1- 20 121 627
- US-A1- 2004 187 871

## Description

The invention relates to a method and device for the operation of an artificial respiration system.

There is described in DE 201 13 789 U1 a medical device with a basic unit, a control unit and at least one additional device connectable to the basic unit so as to be replaceable, whereby the basic unit contains at least one transponder interrogation device for wireless communication with a transponder in the additional device, which communicates information about the additional device to the interrogation device upon request from the latter. The information is used by the basic unit to determine operational parameters for the basic unit.

A similar device is described in WO 02/078775.

For the operation of artificial respiration systems, there is a need for the rapid and, where possible, non-manual retrieval of data into the artificial respiration system which are specific to the respiratory flow sensor and, if need be, specific to the patient and treatment, so that the artificial respiration is adapted to the given specific circumstances.

Accordingly, the problem of the invention consists in providing a method and an accompanying device which quickly make it possible with simple means to operate an artificial respiration system with current operational data relevant to the operation.

The solution to the problem is obtained with the features of claim 1 for the method and with the features of claim 8 for the accompanying device.

The sub-claims provide advantageous developments and configurations of the method according to claim 1.

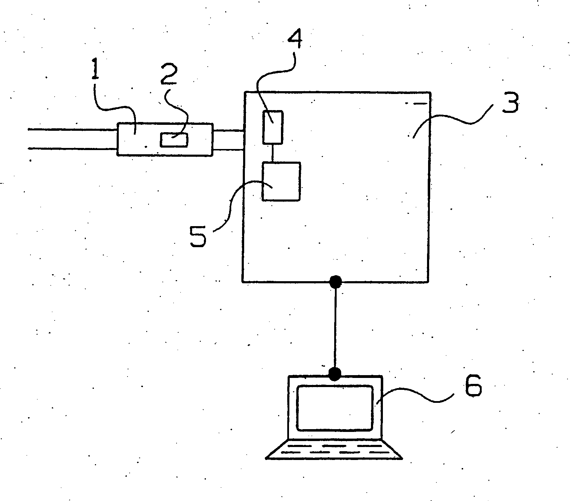

An example of embodiment is explained below with the aid of the single figure, which shows diagrammatically an arrangement for the performance of the method, with a respiratory flow sensor 1 in a respiratory gas line for the artificial respiration of a patient, whereby respiratory flow sensor 1 is specially designed as a hot-wire anemometer known per se and is equipped on the sensor housing with a transponder 2 glued for example on the outside.

Artificial respiration system 3, for example an anaesthesia apparatus, is equipped with a transponder-communication unit 4 with an aerial and with a reading and writing unit 5, which is connected to the operational electronics of artificial respiration system 3, so that operational data of transponder 2 specific to the respiratory flow sensor can in particular be read out and retrieved wireless by artificial respiration system 3 via transponder-communication unit 4 and current operational data of artificial respiration system 3 can be stored in respiratory flow sensor 1. Artificial respiration system 3 is controlled by operator or processor unit 6, for example from a central workplace. If operator or processor unit 6 communicates with a higher-order data information system, the acquired and transmitted operational data can be incorporated into a clinic-wide documentation system or into ordering procedures according to corresponding service times of respiratory flow sensor 1 or operational statuses.

After the start of artificial respiration system 3 via operator or processor unit 6, a specific operating mode for the patient being ventilated by means of artificial respiration system 3 is selected by the operative. Reading and writing unit 5 then takes recourse via transponder-communication unit 4 to patient- and/or treatment-related data and identification data filed in transponder 2 in respiratory flow sensor 1, including for the current quality of the relevant data, of respiratory flow sensor 1 for artificial respiration system 3, so that thereafter all the necessary information is present in artificial respiration system 3 for the artificial respiration to be commenced or optimised.

In the case of unapproved or unacceptable respiratory flow sensors 1, no artificial respiration begins and a suitable alarm or display occurs on artificial respiration system 3.

After the end of artificial respiration, after preset time intervals or in response to an appropriate command from the operative of artificial respiration system 3, current operational data of artificial respiration system 3 are transmitted via reading and writing unit 5 and via transponder-communication unit 4 into the data memory of transponder 2.

Alternatives to the RFID (radio frequency identification) system with an RFID transponder/tag and an RFID reader used in the example are systems with barcodes and optical scanners for the readout, suitable magnetic systems and other wireless inductive or capacitive systems known per se.

The operational data specific to the respiratory flow sensor also include actual release data, which are stored in the data memory of transponder 2 and made available to artificial respiration system 3 upon request. The quality of the sensor measurement data is enhanced by the actual release data if a correction specific to the respiratory flow sensor takes place for the measurement values in accordance with the actual release data, so that for example measurement data precisely defined outside the preset tolerance range for respiratory flow sensor 1 can also be used. The selection of the permissible measurement values is especially important, because they intervene in control circuits of artificial respiration system 3.

## Claims

1. A method for the operation of an artificial respiration system (3), in which a reading unit present in the artificial respiration system (3) reads out wirelessly the contents of a means connected to a respiratory flow sensor (1) which stores operational data for the artificial respiration system (3), **characterized in that** the method is a method for the operation of the artificial respiration system (3) by means of the respiratory flow sensor (1) and the artificial respiration system (3) is ready for operation only after the operational data have been read out, and **in that** the operational data comprise identification data and/or quality data of the respiratory flow sensor (1) for the artificial respiration system (3), which are compared by the artificial respiration system (3) with identification data and/or quality data stored there, and the artificial respiration system (3) is ready for operation only when there is agreement of the compared operational data.

2. The method according to claim 1, in which, after the operation of the artificial respiration system (3), current operational data of the artificial respiration system (3) are transmitted wirelessly each time into the means connected to the respiratory flow sensor (1) for the storage of operational data and the data are stored there.

3. The method according to any one of the preceding claims, in which the operational data comprise patient-related and/or treatment-related data.

4. The method according to any one of the preceding claims, in which the operational data comprise data specific to the respiratory flow sensor, in particular actual release data, so that the measurement values of the respiratory flow sensor (1) to be used in the artificial respiration system (3) are suitably corrected for its operation.

5. The method according to any one of the preceding claims, in which the means connected to the respiratory flow sensor (1) for the storage of operational data is a writable and/or readable transponder (2) and the reading unit present in the artificial respiration system (3) is combined with a writing unit for the transponder (2).

6. The method according to any one of the preceding claims, in which the respiratory flow sensor (1) is a hot-wire anemometer with a sensor housing made from a plastic and the means for the storage of operational data is connected to the sensor housing by means of a bonding agent.

7. The method according to any one of the preceding claims, in which the artificial respiration system (3) is an anaesthesia or artificial respiration device.

8. A device for the operation of an artificial respiration system in accordance with a method according to any one of the preceding claims, in which an artificial respiration system (3) is equipped with a reading unit and a respiratory flow sensor (1) is equipped with an assigned, wireless-readable means for the storage of operational data for the artificial respiration system, wherein the artificial respiration system (3) is adapted to be ready for operation only after operational data stored in the wireless-readable means and comprising identification data and/or quality data of the respiratory flow sensor (1) have been read out wirelessly by the reading unit and have been compared by the artificial respiration system (3) with identification data and/or quality data stored there and when there is agreement of the compared operational data.

9. The device as claimed in claim 8, in which the artificial respiration system includes a writing unit (5).

10. The device as claimed in claim 8 or 9, in which the wireless-readable means is a transponder (2).

## Patentansprüche

1. Verfahren zum Betreiben eines Systems (3) zur künstlichen Beatmung, bei dem eine in dem System (3) zur künstlichen Beatmung vorhandene Leseeinheit drahtlos den Inhalt einer Einrichtung ausliest, die mit einem Beatmungsvolumenstromsensor (1) verbunden ist und Betriebsdaten für das System (3) zur künstlichen Beatmung speichert, **dadurch gekennzeichnet, dass** das Verfahren ein Verfahren zum Betreiben des System (3) zur künstlichen Beatmung mittels des Beamtungsvolumenstromsensors (1) ist und das System (3) zur künstlichen Beatmung nur betriebsbereit ist, nachdem Betriebsdaten ausgelesen worden sind, und dass die Betriebsdaten Identifikationsdaten und/oder Qualitätsdaten des Beatmungsvolumenstromsensors (1) für das System (3) zur künstlichen Beamtung umfassen, die von dem System (3) zur künstlichen Beatmung mit darin gespeicherten Identifikationsdaten und/oder Qualitätsdaten verglichen werden, und dass das System (3) zur künstlichen Beatmung nur betriebsbereit ist, wenn Übereinstimmung der verglichenen Betriebsdaten besteht.

2. Verfahren nach Anspruch 1, bei dem, nach Betrieb des Systems (3) zur künstlichen Beatmung, aktuelle Betriebsdaten des Systems (3) zur künstlichen Beatmung jedes Mal drahtlos in die mit dem Beatmungsvolumenstromsensor (1) verbundene Einrichtung für die Speicherung der Betriebsdaten übertragen und die Daten dort gespeichert werden.

3. Verfahren nach einem der vorhergehenden Ansprüche, bei dem die Betriebsdaten Daten mit Patientenbezug und/oder Daten mit Behandlungsbezug umfassen.

4. Verfahren nach einem der vorhergehenden Ansprüche, bei dem die Betriebsdaten Daten umfassen, die spezifisch für den Beatmungsvolumenstromsensor sind, insbesondere aktuelle Ausgabedaten, so dass Messwerte des Beatmungsvolumenstromsensors (1), die von dem System (3) zur künstlichen Beatmung verwendet werden sollen, für seinen Betrieb geeignet korrigiert werden.

5. Verfahren nach einem der vorhergehenden Ansprüche, bei dem die mit dem Beatmungsvolumenstromsensor (1) verbundene Einrichtung für die Speicherung von Betriebsdaten ein beschreibbarer und/oder auslesbarer Transponder (2) ist und die in dem System (3) zur künstlichen Beatmung vorhandene Leseeinheit mit einer Schreibeinheit für den Transponder (2) kombiniert ist.

6. Verfahren nach einem der vorhergehenden Ansprüche, bei dem der Beatmungsvolumenstromsensor (1) ein Hitzdrahtanemometer mit einem aus Kunststoff hergestellten Gehäuse ist und die Einrichtung zur Speicherung von Betriebsdaten mit dem Sensorgehäuse durch ein Haftmittel verbunden ist.

7. Verfahren nach einem der vorhergehenden Ansprüche, bei dem das System (3) zur künstlichen Beatmung ein Anästhesiegerät oder ein Gerät zur künstlichen Beamtung ist.

8. Vorrichtung zum Betreiben eines Systems zur künstlichen Beatmung gemäß einem Verfahren nach einem der vorhergehenden Ansprüche, bei dem ein System (3) zur künstlichen Beatmung mit einer Leseeinheit ausgerüstet ist und ein Beatmungsvolumenstromsensor (1) mit einer zugeordneten drahtlos auslesbaren Einrichtung für die Speicherung von Betriebsdaten für das System zur künstlichen Beatmung versehen ist, wobei das System (3) zur künstlichen Beatmung dazu eingerichtet ist, nur betriebsbereit zu sein, nachdem Betriebsdaten, die in der drahtlos auslesbaren Einrichtung gespeichert sind und Identifikationsdaten und/oder Qualitätsdaten des Beatmungsvolumenstromsensors (1) umfassen, von der Leseeinheit drahtlos ausgelesen worden sind und durch das System (3) zur künstlichen Beatmung mit darin gespeicherten Identifikationsdaten und/oder Qualitätsdaten verglichen worden sind, und wenn Übereinstimmung zwischen den verglichenen Betriebsdaten besteht.

9. Vorrichtung nach Anspruch 8, bei der das System zur künstlichen Beatmung eine Schreibeinheit (5) enthält.

10. Vorrichtung nach Anspruch 8 oder 9, bei der die drahtlos auslesbare Einrichtung ein Transponder (2) ist.

## Revendications

1. Procédé pour le fonctionnement d'un système de respiration artificielle (3), dans lequel une unité de lecture présente dans le système de respiration artificielle (3) lit, sans fil, le contenu d'un moyen relié à un capteur de débit respiratoire (1) qui mémorise des données opérationnelles pour le système de respiration artificielle (3), **caractérisé en ce que** le procédé est un procédé de fonctionnement du système de respiration artificielle (3) au moyen du capteur de débit respiratoire (1) et le système de respiration artificielle (3) est prêt à fonctionner uniquement après que les données opérationnelles ont été lues, et **en ce que** les données opérationnelles comprennent des données d'identification et/ou des données de qualité du capteur de débit respiratoire (1) pour le système de respiration artificielle (3), qui sont comparées par le système de respiration artificielle (3) avec des données d'identification et/ou des données de qualité mémorisées dans celui-ci, et le système de respiration artificielle (3) ne peut fonctionner que lorsqu'il existe une concordance des données opérationnelles comparées.

2. Procédé selon la revendication 1, dans lequel, après le fonctionnement du système de respiration artificielle (3), les données opérationnelles du moment du système de respiration artificielle (3) sont transmises, sans fil, à chaque fois au moyen relié au capteur de débit respiratoire (1) pour la mise en mémoire des données opérationnelles et les données y sont mémorisées.

3. Procédé selon l'une quelconque des précédentes revendications, dans lequel les données opérationnelles comprennent des données relatives au patient et/ou des données relatives au traitement.

4. Procédé selon l'une quelconque des précédentes revendications, dans lequel les données opérationnelles comprennent des données spécifiques au capteur de débit respiratoire, en particulier des données de sortie réelles, de telle sorte que les valeurs de mesure du capteur de débit respiratoire (1) devant être utilisées dans le système de respiration artificielle (3) sont corrigées de façon appropriée pour son fonctionnement.

5. Procédé selon l'une quelconque des précédentes revendications, dans lequel le moyen relié au capteur de débit respiratoire (1) pour la mise en mémoire des données opérationnelles est un transpondeur inscriptible et/ ou lisible (2) et l'unité de lecture présente dans le système de respiration artificielle (3) est combinée avec une unité d'écriture pour le transpondeur (2).

6. Procédé selon l'une quelconque des précédentes revendications, dans lequel le capteur de débit respiratoire (1) est un anémomètre à fil chaud avec un boîtier de capteur en matière plastique et le moyen de mise en mémoire de données opérationnelles est relié au boîtier de capteur à l'aide d'un agent adhésif.

7. Procédé selon l'une quelconque des précédentes revendications, dans lequel le système de respiration artificielle (3) est un dispositif d'anesthésie ou de respiration artificielle.

8. Dispositif pour le fonctionnement d'un système de respiration artificielle selon le procédé de l'une quelconque des précédentes revendications, dans lequel un système de respiration artificielle (3) est équipé d'une unité de lecture et un capteur de débit respiratoire (1) est équipé d'un moyen lisible sans fil, affecté, de mémorisation de données opérationnelles pour le système de respiration artificielle, dans lequel le système de respiration artificielle (3) est adapté pour pouvoir fonctionner uniquement après que les données opérationnelles mémorisées dans le moyen lisible sans fil et comprenant des données d'identification et/ou des données de qualité du capteur de débit respiratoire (1) ont été lues, sans fil, par l'unité de lecture et ont été comparées par le système de respiration artificielle (3) avec des données d'identification et/ou des données de qualité mémorisées dans celui-ci, et lorsqu'il existe une concordance des données opérationnelles comparées.

9. Dispositif selon la revendication 8, dans lequel le système de respiration artificielle comprend une unité d'écriture (5).

10. Dispositif selon la revendication 8 ou 9, dans lequel le moyen lisible sans fil est un transpondeur (2).
